# EUROPEAN PATENT APPLICATION

(11) **EP 4 364 752 A1**
(43) Date of publication of application: **08.05.2024**
(21) Application number: 22205884.4
(22) Date of filing: 07.11.2022
(51) Int. Cl.: A61K 39/00, C07K 14/47, G01N 33/68

(54) **IMPROVED VACCINE**

(71) Applicant: OncoDNA, 6041 Charleroi (BE)
(72) Inventor: VAN HUFFEL, Christophe, 1332 Rixensart (BE); DETIFFE, Jean-Pol, 6500 Beaumont (BE)
(74) Representative: Calysta NV

(57) **Abstract**

A synthetic DNA molecule comprising one segment encoding a tumor neoantigen or an epitope from an infectious agent under the control of a promoter for the transcription into a corresponding RNA molecule and a segment for the translation of the said translated RNA molecule into a peptide.

## Description

### Technical domain

The present disclosure concerns the use of short epitopes, preferably in a pool, for vaccination.

More specifically, but not exclusively, the present disclosure refers to a process for the production of a DNA molecule able to be transcribed into a RNA vaccine, wherein the encoded epitope is purposedly short.

### State of the art

Document US2015038373A1 provides a method for de novo gene synthesis. In particular, the document provides methods, devices and systems that can quickly synthesize large gene libraries or relatively longer oligonucleotide fragments efficiently with less error. The substrate used for *de novo* gene synthesis is silicon.

The document EP3576751 provides a ribonucleic acid (RNA) cancer vaccine that can safely direct the body's cellular machinery to produce nearly any cancer protein or fragment thereof of interest. This document exemplified a cancer vaccine having 20 epitopes or 52 epitopes. The document describes a mRNA cancer vaccine having an open reading frame encoding a concatemer of epitopes.

The document WO2020/097291 describes a method for treating a patient's cancer with an mRNA vaccine where the vaccine nucleic acid has maximum anti-cancer efficacy for a given length.

The document by Kreiter Sebastian et al. (The Journal of Immunology, Vol 180, 2008, pp 309-318) mentions the efficiency of antigen presentation by coupling antigens to MHC molecules, where the vector described has a promoter, a poly-A tail, and 3' UTR sequences.

The document by Nielsen et al. (Journal of Immunological Methods, Vol 360, 2010, pp 149-156) describes the *in vitro* transcription of a polyepitope mRNA construct encoding 32 epitopes of CMV, EBV and influenza, wherein the mRNA construct contains a DC-LAMP signal sequences, 3'-UTR sequences and a poly-A tail.

The document WO2018/183922 describes a method and compositions for a vaccine against malaria, wherein the vaccine can comprise a cocktail of several antigens produced using plasmids or adenoviral expression vectors.

Unfortunately, although these documents describe the merits of their technology, currently de novo synthesis of DNA molecule, remains a difficulty, as the technical limitation associated to the size of the DNA molecule, when chemically synthetized, remains a concern, whereas long epitopes are preferred to ensure a good loading by the cellular machinery, maximizing the immune response.

On the other hand, the synthesis of a plurality of short DNA molecules, then the correct *in vitro* translation is a challenge. Moreover, there is limited predictability for the *in vivo* response.

Finally, there has been a lot of attempts to perform vaccination using cocktails of short peptidic neoantigens to treat patients affected with cancer (e.g. NCT02897765). However, none of them has been successful, despite showing immunogenicity.

### Summary of the invention

The present invention discloses a synthetic DNA molecule, a pool of synthetic DNA molecules and their corresponding RNAs for use in vaccination as defined in the claims.

### Brief description of the Figures

Figure 1 shows the synthetic DNA and corresponding mRNA sizes where PolyA tails of different size are incorporated by PCR.
Figure 2 shows the correlation between the amount of DNA and the transcribed mRNA.
Figure 3 shows the organization of the multiwell plates of Figure 4.
Figure 4 shows the immune response from control (A, E) and treated mice.
Figure 5 shows the relative abundance of myeloid cells in control and treated mice.
Figure 6 shows the relative abundance of γδ cells and the CD4/CD8 ratios in control and treated mice.
Figure 7 shows the activation of the CD8 cells.

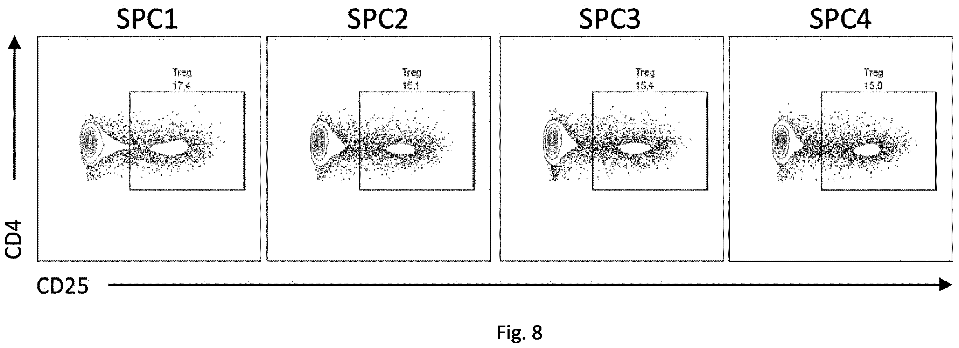

Figure 8 shows the relative abundance of Treg cells.

### Detailed description of the invention

The inventors have developed RNA-based vaccine formulations and, to their surprise, when a pool of RNA molecules with short epitope segment is administered *in vivo,* the immune response is very strong (as compared with concatemers), whereas a dilution would have been expected.

Accordingly, a first aspect of the present invention is a (synthetic) DNA molecule comprising :
- a promoter for the transcription of this synthetic DNA molecule into a corresponding RNA molecule,
- a segment for the translation by a Eukaryotic cell of this transcribed RNA molecule into a peptide and/or for improving the stability within a Eukaryotic cell of the said transcribed mRNA molecule,
- an Open Reading Frame comprising an epitope (for instance in SEQ ID NO:1, the three undefined nucleotides "nnn" at position 159-161, represents the epitope, which is a nucleotide sequence encoding a desired epitope; from 7 to 50 amino acids), and
- a 3' Poly A tail segment;
this epitope ("nnn" in SEQ ID NO:1), when translated, having a size between 7 and 50 amino acids, and/or this Open Reading Frame having a size between 21 nucleotides and 500 nucleotides, preferably between 30 and 400 nucleotides, still more preferably between 42 and 330 nucleotides. The inventors have succeeded in using an Open Reading Frame of a size between 300 and 330 nucleotides.

This allows to keep a distance between the start codon (ATG) and the poly A tail as short as reasonably possible, and to take benefit of two addressing elements, for an improved cellular processing.

Indeed, as it will be shown in the Examples, such short mRNA are extremely well translated (*in vivo*)*,* allowing to mix them (or to mix the corresponding synthetic DNA) to form a pool: even after the dilution in the pool, the immune response to the epitopes is unexpectedly robust: proportion of the epitope triggering an immune response, level of the response, quality of the response.

In the context of the present invention, preferably, the encoded epitope has a size between 7 and 50 amino acids, preferably between 8 and 45 amino acids, more preferably a size between 12 and 40 amino acids, still more preferably a size between 16 and 35 amino acids, such as between 20 (or 21, 22, 23, 24, 25) and 31 (or 30, 29, 28, 27) amino acids. These epitopes can be designed with flanking residues, at their C or N terminus, for instance one or two amino acids, such as glycine. When the epitope does not comprise a signal peptide nor an endosomial addressing sequence, the epitope comprises a methionine residue at its N terminal, either because the epitope as designed (from its target) comprises a methionine, or because a methionine is added at the N terminal flanking residue.

These epitopes can be designed, starting from a target (peptide from an infectious agent, neoantigen; peptide causing an unwanted immune response in a patient), for a preferred loading by MHC Type I (sequence prediction and the design of an epitope of a minimal size of 7 amino acids), MHC Type 2 (sequence prediction and the design of a minimal size of 12 amino acids), or by other presentation systems, including the CD1(d) molecule of the NKT cells (prediction; minimal size of 7 amino acids). Advantageously, such short epitopes, while potentially designed for the loading by one class of receptor of immune cells (e.g. MHC, Type I), have conversely a low probability of being loaded by a different class of receptor of immune cells (e.g. MHC, Type 2).

Preferred promoters are from a viral origin, and/or generate mRNA with a 5'end Cap structure, such as the T7 promotor and/or sequences sharing at least 93% of identity with the segment between nucleotides 1 and 15 of SEQ ID NO:1.

Preferably, the Open Reading Frame of this synthetic DNA molecule comprises a segment being an endosomal signal sequence having a size between 60 and 99 nucleotides and/or this segment shares at least 95% of identity with the segment between nucleotides 66 and 158 of SEQ ID NO:1 and/or this segment after translation shares at least 95% of identity with the encoded corresponding peptide.

This endosomal signal sequence segment is in-frame with this epitope. However, in the above-mentioned sizes (size of the epitope), this endosomal signal sequence is not part of the epitope.

This sequence element addresses the epitope in the endosomes, possibly in proximity to nascent MHC Type I molecules (or MHC, Type 2 molecules), which will advantageously increase the probability of being loaded by an MHC Type I (or Type 2) molecule.

Conversely, when humoral responses are desired, this sequence element can be omitted or a different peptide signal sequence is preferred, so as to no longer address the epitope in the proximity to nascent MHC Type I molecule. When no signal sequence is present, an ATG codon (methionine) needs to be added, to launch the translation.

Preferably, the Open Reading Frame of this synthetic DNA molecules further comprises a (second) segment (in-frame with the epitope, preferably downstream the epitope) for addressing the encoded epitope to MHC molecules, such as nascent MHC Type I molecule. Preferably this segment has a size comprised between 60 and 300, preferably between 90 and 120 nucleotides and/or this segment shares at least 95% of identity with the segment between nucleotides 162 and 284 of SEQ ID NO:1 and/or this segment shares at least 95% of identity with the encoded corresponding peptide and/or this segment shares the same function as the peptide encoded by the segment between nucleotides 162 and 284 of SEQ ID NO:1.

Despite the increase of the size of the construct and the corresponding difficulty to keep a short distance between the start codon and the poly A tail, the inventors have found that the incorporation of such sequence elements boosts the (*in vivo*) response to the epitope, being advantageously a CD8 response.

On the other hand, again, when other types of immune responses are preferred, this sequence element is advantageously omitted.

Advantageously the synthetic DNA molecule further comprises a segment for (improving) the translation being at the 5' of (i.e. before) the epitope (and/or Open Reading Frame) and/or a segment for improving the stability within a Eukaryotic cell of the said transcribed mRNA molecule, which is at the 3' (downstream) of this Open Reading Frame.

Again, despite the increased length, which is problematic, especially when placed at the 3'-end, the inventors have noticed a more robust response when this element has been added.

Preferably, this segment for improving the stability within a Eukaryotic cell of the said transcribed mRNA molecule, which is at the 3' of this Open Reading Frame, has a size comprised between 50 and 200 nucleotides, more preferably between 100 and 150 nucleotides and/or this segment shares at least 95% of identity with the segment located between nucleotides 285 and 419 of SEQ ID NO:1.

Preferably, the 5' segment for (improving) the translation has a size between 10 and 100 nucleotides, preferably between 20 et 75 nucleotides and/or shares at least 95% of identity with the segment spanning between nucleotides 16 and 65 of SEQ ID NO:1.

Preferably the 3'Poly A tail segment comprises at least 15 consecutive adenosine residues. Preferably, the 3' Poly A tail segment has a size of at least 50 nucleotides, preferably of at least 70 nucleotides, such as at least about 100 nucleotides and/or comprises more than 90% of adenosine residues (preferably more than 95% of adenosine residues). Indeed, Poly-A tails are either engineered using only adenosine residues, yet, alternative, poly A tails can be segmented and are still working.

In other words, preferably this 3' Poly A tail segment consists of 100% of adenosine, or
consists essentially of adenosine; for instance the poly A tail segment consists of (i) stretches of at least 10 consecutive adenosine residues, preferably at least 20, 30, 40 consecutive adenosine residues, (ii) separated by other nucleotides (a stretch of less than 20, preferably less than 10 residues, preferably less than 5 residues not forming the above stretch), and (iii) followed by a second stretch of at least 10 consecutive adenosine residues (independently of the length of the first stretch, preferably at least 20, 30, 40 consecutive adenosine residues), possibly followed again by separating nucleotides (less than 20, preferably less than 10, less than 5 residues not forming an homo-adenosine stretch) and subsequent adenosine stretches. In other words, segmented poly adenosine stretches are also comprised by the terminology 'Poly A segment', provided that at least 50, preferably at least 90 adenosine residues, or even preferably at least 120 adenosine residues are present in these stretches, to form the 'Poly A tail segment'.

Advantageously, in this synthetic DNA molecule, the Open Reading Frame and the 3' segment for improving the stability within a Eukaryotic cell of the transcribed mRNA molecule, bridging the Open Reading Frame and the 3' PolyA tail segment, have together a size comprised between 200 and 700 nucleotides, preferably between 300 and 600 nucleotides, more preferably between 400 and 500 nucleotides.

As noticed here above, the inventors have found, surprisingly, that such short segments result into an improved (*in vivo*) translation of the encoded epitope, as well as an improved (intra)cellular targeting.

Preferably, the synthetic DNA molecule of the present invention is not identical to any one of the SEQ ID NO:2 to 61.

Preferably, the synthetic DNA molecule of the present invention (while being different of SEQ IDs Nos 2 to 61) shares similarities with SEQ ID NO:1 and comprises one epitope ("nnn"; hence the triplet "nnn" does not encode only one amino acid, but has a size as defined here below) encoding a peptide having a size between 7 and 50 amino acids, preferably between 8 and 45 amino acids, more preferably a size between 12 and 40 amino acids, still more preferably a size between 16 and 35 amino acids, such as between 20 (or 21, 22, 23, 24, 25) and 31 (or 30, 29, 28, 27) amino acids, and a Poly A-tail and

one or several segments, selected from:
a promoter region sharing more than 93% of identity with the full length of the segment of the nucleotides 1 to 15 of SEQ ID NO:1and/or
a translation enhancer region sharing more than 95% of identity with the full length of the segment of the nucleotides 16 to 65 of SEQ ID NO:1and/or
an endosomal targeting region sharing more than 95% of identity with the full length of the segment of the nucleotides 66 to 158 of SEQ ID NO:1 and/or
a sorting and/or trafficking and/or presentation to T cells region (downstream the epitope) sharing more than 95% of identity with the full length of the segment of the nucleotides 162 to 284 of SEQ ID NO:1and/or
3' enhancer region (mRNA stabilizing region; downstream the epitope or the sorting region) sharing more than 95% of identity with the full length of the segment of the nucleotides 285 to 419 of SEQ ID NO:1.

Preferably, when a cytotoxic response and/or cellular and/or the loading by nascent MHC Type I class is to be elicited, all the above elements are present.

Conversely, when a humoral response is preferred, the sorting sequence element is advantageously omitted, but the promoter and enhancer elements are still incorporated in the construct. A peptide signal sequence, ora codon fora flanking N-terminal methionine, or even the endosomal targeting region is advantageously present.

A related aspect of the present invention is a pool of at least 10, preferably at least 15, preferably at least 20 or at least 50 DNA molecules as above.

Indeed, the inventors have shown how to rapidly synthetize large amounts of such DNA molecules, for instance relying on a common antisense segment.

Such a pool is very useful for vaccination purposes as the inventors have shown a very robust immune response, despite the dilution effect: dilution of the molecules upon mixing, competition during the i*n vitro* translation competition for the cellular effectors involved in the immune response. The inventors have found that the possibility to elicit a desired immune response, which is built epitope-by-epitope (or even peptide sequence by peptide sequence), allows several advantageous features, such as:
the fine-tuning of the immune response, for instance a better control on a cellular (NK cells, NKT epitopes, CD8 cytotoxic T-cells, NK cells, NKT epitopes) or on an humoral response;
the possibility of acting on the immune system for boosting a desired response, or repressing a deleterious response...);
increased options to avoid potential escape mutations of the target, or even to already administer an epitope targeting a predicted escape mutation (e.g. for treating AIDS or rapidly mutating cancers);
the flexibility to use any peptide sequences, even not favored upon bio-informatic analysis.

Moreover, the option to use short epitopes will avoid the potential problem that may occur where a mRNA encoding a long epitope is injected to a patient. This risks to trigger unwanted responses: when translated by a cell of a patient, at the cellular level, at the organ level, or even at the patient level, since the peptide is synthetized in cells with limited type-predictability and at locally unknown levels. Such a peptide, especially if able to bind to cellular effectors, can trigger unwanted secondary effects. The present inventors have found that short epitopes of the present invention overcome this.

The inventors consider that the ability to elicit a CD8 response, or a non-CD4 response, is advantageous for cancer therapies and also for infectious diseases. For instance, in AIDS-affected patients, the pool of CD4 cells is reduced, but the amount of CD8 cells is still maintained: a specific vaccine triggering a robust cytotoxic response is therefore a therapeutic tool to specifically attack the reservoirs of such virus.

In other words, the possibility to elicit non-CD4 responses, including cytotoxic responses or even through NKT cells opens new approaches for vaccination.

In the context of the present invention, preferably, the wording "elicit a CD8 response" refers to a relative abundance of the CD8 T cells in a sample (number of the CD8 T cells:total number of the T cells) increased (not treated sample vs treated sample) by at least 10%, preferably by at least 20%, more preferably by at least 30% or even by at least about 50%, and/or by a proportion of CD8 T cells expressing CD44 marker increased (CD8 T cells of a not treated sample vs CD8 T cells of a treated sample) by at least 10%, preferably by at least 20%, more preferably by at least 30% (advantageously, the proportion of CD4 T cells expressing CD44 does not show an increase of 10% or more in the same settings and sample). The co-expression of CD8 (or CD4) and CD44 levels, in this test, is preferably measured by Fluorescence Associated Cell Sorting (FACS).

In this test, preferably, the sample is a from a body fluid, or from an affected (*ex vivo)* tissue (biopsy of a tissue infected by an infectious agent, tumor biopsy).

The same definition advantageously is applicable for vaccination aiming at increasing the level or the activity of CD4 cells or even for inducing specific Treg cells (the proportion of CD4 cells expressing CD25).

Advantageously all the DNA molecules of this pool share the same promoter for the transcription, being preferably a promoter sharing more than 93% of identity with the full length of the segment of the nucleotides 1 to 15.

The choice of the same promoter allows a simplified *in vitro* transcription.

Preferably, the pool of synthetic DNA molecules does not comprise all the sequences SEQ ID NO:2 to 61, more preferably comprises less than 90% (less than 80%, less than 50%, less than 20%, less than 10% or even none) of the sequences SEQ ID NO:2 to 61.

Another related aspect of the present invention is a RNA (a mRNA) molecule obtained upon transcription of the above DNA molecule.

Preferably, the transcribed RNA comprises a 5' Cap (see below in the translation process).

A closely related aspect of the present invention is a pool of RNA (mRNA) molecules obtained upon transcription of the above pool of the DNA molecules.

Preferably, this (m)RNA molecule or the (m)RNA molecules of this pool comprise modified nucleotides. Pseudouridine and/or of N1-Methylpseudouridine, 15-methyluridine (m5U), and/or 2-thiouridine (s2U) are among the advantageous modifications. These nucleotide analogs are advantageously incorporated during the transcription, using nucleotide mixes with such analog, possibly at the expense of the uridine content.

This increases the mRNA stability and/or boosts the immune response and/or reduces the toxicity of the mRNA upon injection to a patient.

Another related aspect of the present invention is a vaccine composition comprising this pool of the (m)RNA molecules.

This vaccine is advantageous against infectious diseases, for instance with epitopes targeting different infectious agents, and/or different epitopes present on different proteins from one infectious agent.

This vaccine is similarly advantageous against cancer, wherein at least 10%, preferably at least 20%, more preferably at least 50% of the identified neoepitopes are covered by the vaccine.

When the vaccine is to be used against a solid tumor, the status of the tumor and/or the capacity of the immune system to attack it is advantageously checked and, if needed, anti PDL1 (or PD1) therapies are advantageously considered together with the present vaccine. Other molecules boosting the immune system are also advantageously used in synergy with the vaccine of the present invention, especially in a patient having a depressed immune response. Preferably, chemotherapeutic treatments affecting the immune response are stopped before the administration of the vaccine of the present invention (or replaced by another chemotherapeutic regimen, allowing an increased immune response), or at least the level of the immune response is monitored and considered as sufficient before administration of the vaccine of the present invention. On the other hand, since the inventors have shown the potential to elicit a very robust and specific CD8 cytotoxic response, the problems associated to a potential downregulation of the immune system in a patient, or in the tumor microenvironment are reduced.

Conversely, this vaccine is also advantageous to turn-off an unwanted immune response.

This vaccine is also advantageous, as above-mentioned, to selectively boost an immune cellular response or, conversely, to selectively boost an immune humoral response.

In other words, this vaccine is advantageously to elicit CD4+ and/or CD8+ T cells specific for the encoded epitopes. This is especially advantageous for patients having an unbalanced immunity, for instance towards infectious diseases, cancer, or inflammatory diseases. Especially the generation (or restauration) of a strong cellular (cytotoxic) response is, as above described, a clear advantage disclosed by the present invention.

Another related aspect of the present invention is a process to obtain a vaccine composition comprising the step of obtaining a pool of synthetic DNA molecules, to perform *in vitro* the transcription into (m)RNA of the DNA molecules comprised in this pool and to formulate the (m)RNA molecules into a vaccine composition.

Preferably, this process allows the addition of a 5' Cap to the transcribed (m)RNA, this Cap being preferably a sequence comprising two nucleotides linked in 5'-3' by a triphosphate stretch (preferably G-PiPiPi-N1, the first nucleotide to be transcribed) and/or comprising a 2'-O-Me residue.

Several polymerases, such as the T7 polymerase, start with such a Cap. Accordingly, the mix for *in vitro* transcription preferably comprises the starting molecule able to form the Cap, advantageously the so-called Anti-Reverse Cap Analog, which preferably comprises a 3'O-Me modification to favor the correct orientation (i.e. mG-PiPiPi-N1; N1 being the first nucleotide of the translated mRNA, if uncapped). In a subsequent step, this 3'O-Me is advantageously (enzymatically) removed.

The 5' Cap can thus be inserted during the transcription step.

Alternatively, the 5' Cap can be grafted on the formed mRNA in a separate capping step, for instance using the Vaccinia capping system together with GTP and S adenosine methionine.

In these two alternatives, a further 2'O methylation near the Cap is advantageous, which is preferably achieved enzymatically, advantageously using a Cap 2'-O-Methyltransferase and S-adenosine methionine.

Preferably, this process further comprises the step of verifying the integrity of the obtained mRNA.

The inventors have noticed that the full-length mRNAs obtained through the settings of the present invention are much better translated (*in vivo)* by the patient's cells. In the above settings with short DNA constructs, the inventors have noticed a very large proportion of full-length (m)RNA. Yet, conversely, this implies that longer constructs, or constructs more difficult to translate, for instance due to secondary structures and/or specific sequences, risk sometimes to be transcribed at a lower efficiency. Also, when the Poly A tail is incorporated by PCR (see below), the inventors have noticed an higher proportion of errors. This explains the advantage of this additional step.

More into details, the (synthetic) DNA molecules of this pool are obtained as described above, by combining (upon chemical synthesis) 5' elements, optionally (but preferably) a coding sequence targeting an epitope to the endosome, a coding sequence (in-frame with the optional endosomal targeting sequence) being the epitope, optionally a sequence for the correct trafficking of the epitope(also in-frame with the epitope), optional 3' untranslated elements (e.g. stabilization) and a poly A tail. This approach is described, for instance in PCT/EP2022/075375 and/or in PCT/EP2022/075374, both filed on September 13, 2022.

Since the chemical synthesis of very long DNA molecules (for instance of about 600 nucleotides) is still a challenge, and not possible in practice if a large amount of different long molecule needs to be synthetized to treat one patient, the synthesis can advantageously be performed in a multi-step scheme, where (i) the 5' (truncated) part of the (final) DNA molecule is synthetized as a sense molecule, (ii) the 3' (truncated) part of the (same strand of the final) DNA molecule is synthetized as an antisense molecule, both sense and antisense molecules presenting an overlap big enough to allow pairing and an *in vitro* elongation, and (iii) the elongation of the two 'truncated' strands, so as to form a regular double stranded molecule, each truncated strand acting as the template for the other strand. In this scheme, no specific ligation step is performed.

Advantageously, the antisense strand comprises trafficking and/or stabilizing elements (including the Poly A tail), 3' of the epitope, which are segment that are advantageously common for several synthetic constructs.

Advantageously, the antisense strand is thus common to all the constructs (same trafficking and/or stabilizing elements), which simplifies the synthesis procedure. Alternatively, different cellular trafficking sequences can be used, depending on the intracellular processing intended for a given peptide. In this case, the synthesis scheme will be more complex.

In this process, possibly, the poly A tail is added on the (elongated) double stranded DNA in a subsequent step by PCR, instead of being part of the antisense strand described here above), relying on a sense template, for instance being derived from the most 5' extremity, preferably the (common) promoter (such as the T7) and on an antisense template, being a PolyT having further a 3' fragment complementary to the other extremity of the double stranded DNA.

This optional step is not needed in the preferred approach described here above and in which truncated sense and antisense strains are synthetized then elongated (see also in PCT/EP2022/075374). However, if longer elements need to be incorporated, or if a synthesis in one single step is preferred (e.g. different 3' trafficking sequences in the pool), or if the sequence to synthetize can be achieved without relying on the antisense strand, except of a part of the molecule, the possibility to "graft" the Poly A tail through PCR remains advantageous, especially for Poly A tails shorter than 100 consecutive A residues or for segmented Poly A tails (see above). Indeed, the inventors have noticed an higher proportion of errors associated to such mode of incorporation of a long (homo) Poly A tail.

This pool of epitope (mRNA vaccine) is advantageously administered to the patient by inhalation, subcutaneous injection, or intramuscular injection ; Intravascular administration is also possible, if the right encapsulating lipids (i.e. lipids designed for intravascular administration of nucleic acids) are selected.

### Examples

### Example 1: Starting material

The inventors have engineered 96 synthetic DNA molecules and mRNA vaccines by *in vitro* transcription (all the DNA molecules pooled in a single tube) as in PCT/EP2022/075375 and/or in PCT/EP2022/075374, both filed on September 13, 2022.

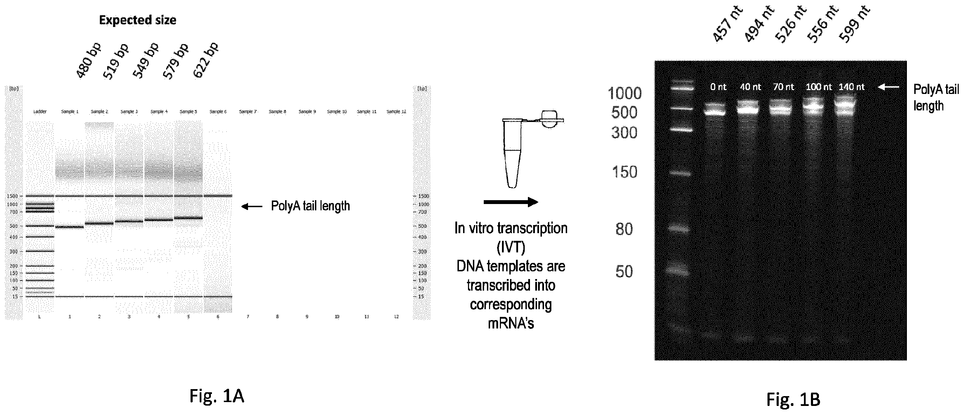

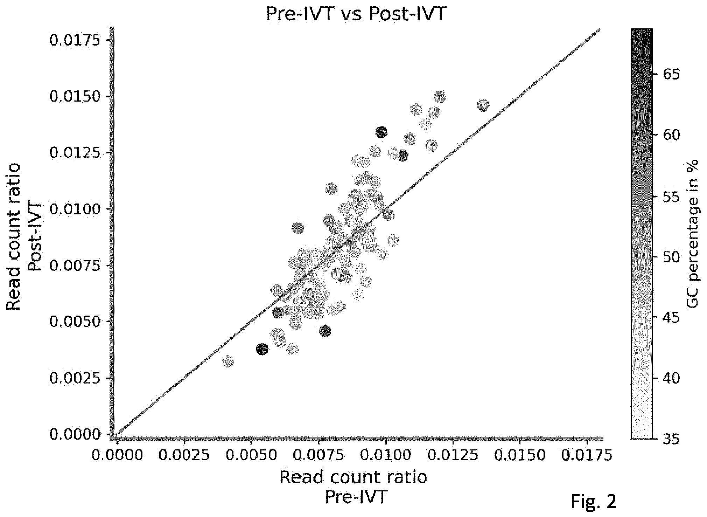

To do so, firstly, the inventors have tested different Poly A tails (40, 70, 100 and 140 nucleotides). The inventors have verified by electrophoresis that the DNA molecules harbor the correct expected size (Figure 1A). Similarly, the corresponding mRNA have the expected size (Fig. 1b). Moreover, there is a good correlation between the abundance of the DNA and the obtained mRNA upon *in vitro* transcription (IVT), meaning an absence of IVT-bias (Figure 2). All the synthetized DNA molecules incorporated (diluted) in the pool have been correctly transcribed.

The pool of mRNA is then formulated with clinically-validated (for intramuscular injection) cationic lipids to form lipidic nanoparticles (LNP); these lipids have been approved for clinical use, for instance in the COMIRNATY^{®} vaccine.

### Example 2 administration to mice

Female Balb/CJ mice (24) have been selected, and injected (Gastrocnemius) at Days 0 and 7, before analysis at Day 21. Body temperature, weight and clinical examination are performed once a week.

Spleen and lymph node extracts have been obtained for further analysis.

The mice were sorted in 4 groups (6 mice per group) buffer injection; 0.1 µg of the mRNA pool in the LNP composition; 1µg of mRNA pool in the LNP composition and 5 µg of the mRNA pool in the LNP composition. In these compositions, the inventors have combined the mRNA pools with a molar excess of lipids of 8 to 13 times, so as to form nanoparticles having a diameter of about 100 nm.

The inventors have noticed an increase in temperature on Days 1 and 8 (1 day post-injection, in the 4 conditions, but more marked for group 4 at Day 8). All the animals gain weight.

### Example 3 - immune response

The first reading was a Fluorospot (readout IFNγ/IL-2 and TNFα), where spleen or lymph node cells from the mice are plated in vitro then challenged with the peptides corresponding to the epitopes, and the level of cytokines production is measured. In practice, since the epitopes are quite long and sometimes not soluble enough, the inventors have used three 15 aminoacids peptides per epitope: one starting at the N terminal; one ending at the C terminal and one spanning in the intermediate region.

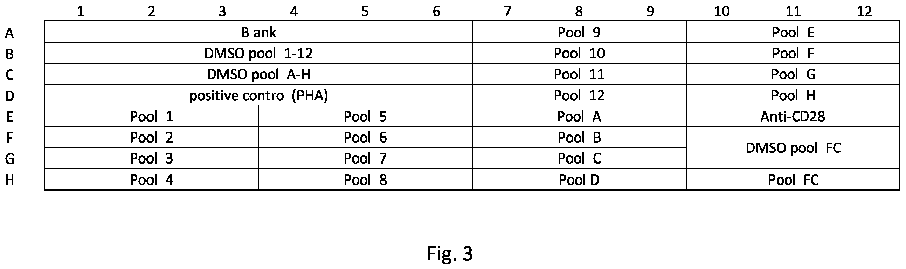

In this preliminary experiment, readout solutions have been firstly organized on multiwells, then pooled (the pools A-H being the lines A1 to A12, B1 to B12, C1 and C12,... H1 and H12, and the pools 1-12 being the columns A1 to H1, A2 to H2,...A12 to H12) and added to the cells according to the scheme of Figure 3 on a specific multiwell comprising sample from lymph node or the samples from spleen cells.

For the spleen cell samples and for the lymph node samples, 200000 cells, have been seeded per well, including for the controls. The negative controls are solutions without the peptides and the positive controls are peptides known to trigger high immune responses in mice.

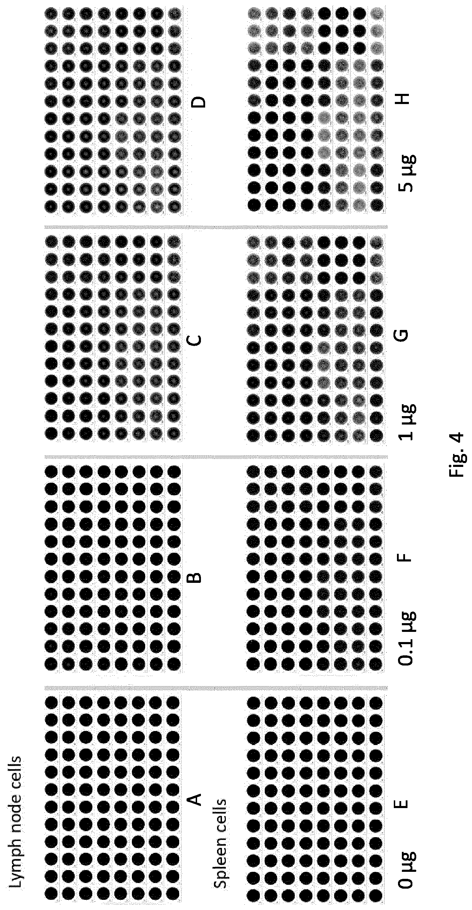

This results into extremely high responses for almost all the pools (Figure 4), meaning that the inventors have succeeded in (i) achieving correct synthesis of a plurality of DNA constructs encoding one epitope each (ii) correctly transcribing *in vitro* this pool so as to formulate a vaccine with a plurality of different epitopes. Moreover, the inventors notice that the response was extremely high, whereas a reduction would have been expected. Beside the level of the response, its quality was also unexpected since all the pools were positive in these settings, both in lymph node and spleen samples. Note that the raw data for the 0.1 µg condition display positive values in the same wells as for the 1 µg and the 5 µg conditions, which are not easily visible at Figure 4. And is dose dependent/follows the higher dose -higher signal correlation.

### Example 4 - characterization of the immune response

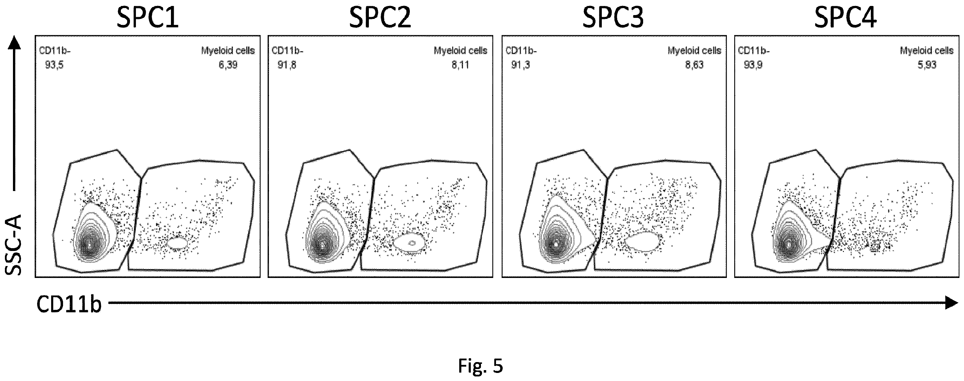

The proportion of myeloid cells remains constant, regardless the mRNA amount (SPC1 being 0; SPC2 being 0.1 µg; SPC3 being 1 µg and SPC4 being 5 µg). See Figure 5.

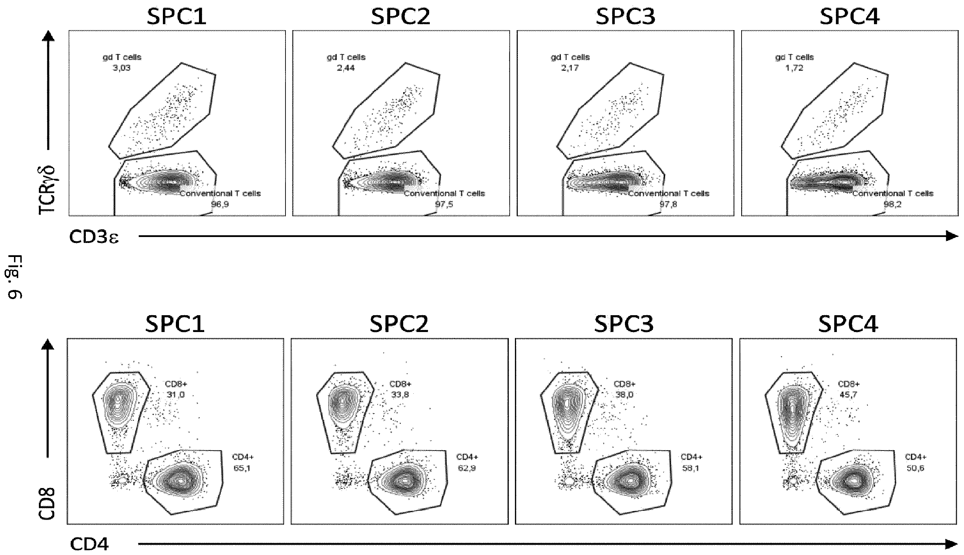

On the other hand, these constructs increase the level of classical αβ TCR cells (Figure 6, upper panel, by subtraction of the γδ level), and the level of the Cytotoxic CD8 cells (Figure 6, lower panel), which is very useful to treat tumors and infected cells.

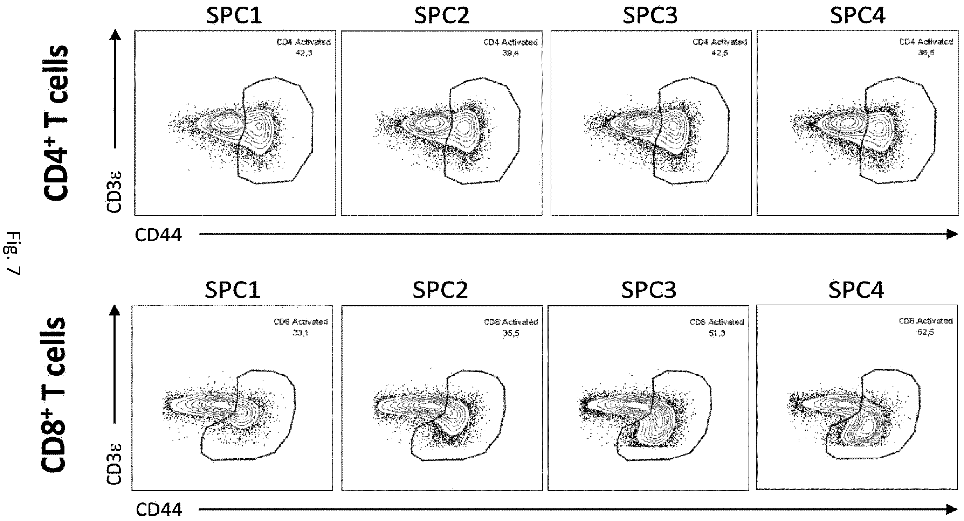

Moreover, the activity level of these CD8 cells is increased by about 50% (Figure 7, lower panel), whereas the activity level of the CD4 cells remains constant.

Similarly, and fortunately in these settings, the activity level of the Treg is not increased.

## Claims

1. A synthetic DNA molecule comprising :
- a promoter for the transcription of the said synthetic DNA molecule into a corresponding RNA molecule,
- a segment for the translation by a Eukaryotic cell of the said transcribed RNA molecule into a peptide and/or for improving the stability within a Eukaryotic cell of the said transcribed mRNA molecule,
- an Open Reading Frame comprising an epitope, and
- a 3' Poly A tail segment,
the said segment for the translation being at the 5' of the said Open Reading Frame and/or the said segment for improving the stability within a Eukaryotic cell of the said transcribed mRNA molecule being at the 3' of the said Open Reading Frame;
the said 3'Poly A tail segment comprising at least 15 consecutive adenosine residues,
the said epitope, when translated, having a size between 7 and 50 amino acids, and
the said Open Reading Frame having a size between 21 nucleotides and 500 nucleotides.

2. The synthetic DNA molecule of claim 1 wherein the Open Reading Frame further comprises a segment targeting and/or trafficking the epitope in a vesicular compartment and/or a sequence for addressing the encoded epitope to MHC molecules.

3. The synthetic DNA molecule of claim 1 or 2, wherein the 3' Poly A tail segment has a size of at least 50 nucleotides, preferably of at least 70 nucleotides, such as at least about 100 nucleotides and/or comprises more than 90% of adenosine residues.

4. The synthetic DNA molecule according to any one of the preceding claims, wherein the Open Reading Frame and the 3' segment for improving the stability within a Eukaryotic cell of the transcribed mRNA molecule, bridging the said Open Reading Frame and the 3' PolyA tail, have together a size comprised between 200 and 700 nucleotides, preferably between 300 and 600 nucleotides, more preferably between 400 and 500 nucleotides.

5. The synthetic DNA molecule according to any one of the preceding claims being not according to any one of the SEQ ID NO:2 to 61.

6. The synthetic DNA molecule according to any one of the preceding claims comprising one or several segments sharing more than 93% of identity with the full length of the segment of the nucleotides 1 to 15 and/or a segment sharing more than 95% of identity with the full length of the segment of the nucleotides 16 to 65 and/or with the full length of the segment of the nucleotides 66 to 158 and/or with the full length of the segment of the nucleotides 162 to 284 and/or with the full length of the segment of the nucleotides 285 to 419 of SEQ ID NO:1.

7. A pool of at least 10, preferably at least 15, preferably at least 20 or at least 50 or at least 80 DNA molecules according to any one of the preceding claims.

8. The pool wherein all the DNA molecules share the same promoter for the transcription, being preferably a promoter sharing more than 93% of identity with the full length of the segment of the nucleotides 1 to 15.

9. A RNA molecule obtained upon transcription of the DNA molecule according to any one of the preceding claims 1 to 6, or a pool of RNA molecules obtained upon transcription of the pool of the DNA molecule of the claims 7 or 8.

10. The RNA molecule or a pool of RNA molecule of claim 9, wherein the said RNA molecule(s) comprise modified nucleotides and/or a 5' Cap.

11. A vaccine composition comprising the pool of the RNA molecules of claim 9 or 10.

12. The vaccine composition of claim 11 for use in eliciting a cytotoxic (CD8) response towards a tumor or towards an infectious agent.

13. A process to obtain a vaccine composition comprising the step of obtaining a pool of DNA molecules according to the claims 7 or 8, to perform *in vitro* the transcription into RNA of the DNA molecules comprised in the said pool and to formulate the RNA molecules into a vaccine composition.

14. The process of claim 13 comprising the step of selecting an enzyme and/or a RNA polymerase adding a 5' Cap to the transcribed RNA, the said 5'Cap being preferably a sequence comprising two nucleotides linked in 5'-3' by a triphosphate stretch.

15. The process of claim 13 or 14 further comprising the step of verifying the integrity of the obtained mRNA.
